# EUROPÄISCHE PATENTSCHRIFT

(11) **EP 2 399 619 B1**
(45) Veröffentlichungstag und Bekanntmachung des Hinweises auf die Patenterteilung: **05.08.2015**
(21) Anmeldenummer: 11168167.2
(22) Anmeldetag: 31.05.2011
(51) Int. Cl.: A61L 31/02, A61L 31/08, A61L 31/14, A61L 31/18

(54) **Implantat und Verfahren zur Herstellung desselben**
Implant and Method for Manufacturing Same
Implant et son procédé de fabrication

(30) Priorität: 25.06.2010 US 358425 P
(43) Veröffentlichungstag der Anmeldung: 28.12.2011
(73) Patentinhaber: Biotronik AG, 8180 Bülach (CH)
(72) Erfinder: Bayer, Ullrich, 18211 Admannshagen-Bargeshagen (DE); Schultz, Wolfgang, 18147 Rostock (DE); Venohr, Nils, 18198 Kritzmow (DE); Riedmüller, Johannes, 90411 Nürnberg (DE); Schröder, Martina, 18184 Kösterbeck (DE)
(74) Vertreter: Galander, Marcus

(56) Entgegenhaltungen:
- EP-A2- 2 184 038
- EP-A2- 2 198 899
- WO-A1-2004/089247

## Beschreibung

Die vorliegende Erfindung betrifft ein Implantat, insbesondere eine intraluminale Endoprothese, mit einem Grundkörper, der zumindest teilweise aus einem metallischen Material besteht, und mit einem an dem Grundkörper befestigten Funktionselement, das zumindest in einem Teil seines Volumens eine verglichen mit dem Material des Grundkörpers verschiedene Materialzusammensetzung aufweist, die vorzugsweise radioopakes und/oder röntgenopakes Material umfasst, sowie ein Verfahren zur Herstellung eines solchen Implantats.

Stents sind endovaskuläre Prothesen (Endoprothesen) oder Implantate, die zur Behandlung von Stenosen (Gefäßverengungen) eingesetzt werden können. Sie weisen meist einen Grundkörper in Form eines hohlzylinder- oder röhrenförmigen Grundgitters auf, das an beiden Längsenden der Röhren offen ist. Das röhrenförmige Grundgitter einer derartigen Endoprothese wird in das zu behandelnde Gefäß eingesetzt und dient dazu, das Gefäß zu stützen. Weitere Grundkörperformen sind ebenfalls möglich. Ferner betrifft die vorliegende Erfindung Implantate, welche im Bereich der Orthopädie verwendet werden können, z.B. für den Schädelbereich, und insbesondere Implantate, die auf Grund ihrer geringen Größe und Wanddicke eine geringe Röntgensichtbarkeit aufweisen.
Die Erfindung kann ebenfalls für Stents im neurovaskulären Bereich zur Anwendung kommen. Hier kommt es darauf an, die hirnversorgenden Blutgefäße mit absorbierbaren Mg-Stents offen zu halten. Diese Systeme kommen auf dem Gebiet der Verhinderung akuter ischämischer Schlaganfälle zum Einsatz.

Stents oder andere Implantate weisen in ihrem Grundkörper häufig metallische Materialien auf. Hierbei können die metallischen Materialien einen biodegradierbaren Werkstoff bilden, wobei auch polymere, biodegradierbare Materialien enthalten sein können.

Unter Biodegradation werden hydrolytische, enzymatische und andere stoffwechselbedingte Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile des Implantats führen. Synonym zu dem Begriff Biodegradation wird häufig der Begriff Biokorrosion verwendet. Der Begriff Bioresorption umfasst die anschließende Resorption der Abbauprodukte durch den lebenden Organismus. Das Ziel der Verwendung biodegradierbarer Implantate besteht darin, dass sie zu einem Zeitpunkt, wenn sie beispielsweise in Bezug auf ihre Stützwirkung nicht mehr benötigt werden, vom Organismus abgebaut sind und demzufolge nicht länger als nötig als Fremdkörper im Organismus vorhanden sind.

Für den Grundkörper biodegradierbarer Implantate geeignete Werkstoffe (Grundmaterial) können aus einem Material oder mehreren Materialien bestehen. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren auf Legierungen von Magnesium, Eisen, Zink und/oder Wolfram.

Die vorliegende Erfindung bezieht sich auf Implantate mit einem Grundkörper, dessen Material einen metallischen Werkstoff enthält. Hierbei kommen neben den genannten biodegradierbaren Materialien auch weitere metallische Werkstoffe in Frage. Insbesondere bezieht sich die vorliegende Erfindung auf Implantate mit dem Bestandteil Magnesium, das vorzugsweise den Hauptbestandteil des Werkstoffs des Grundkörpers bildet.

Die Ermittlung der Position eines Stents oder anderer Implantate geschieht häufig mittels bildgebender Verfahren, beispielsweise mittels einer Röntgenstrahleinrichtung. Auf Grund der kleinen Ordnungszahl und der geringen Dichte des biodegradierbaren Materials Magnesium und seiner Legierungen ist die Röntgensichtbarkeit der daraus gefertigten medizinischen Implantate sehr gering. Um diesen Nachteil zu beheben, ist es bekannt, medizinische Vorrichtungen mit Funktionselementen zu versehen, die zumindest in einem Teil ihres Volumens eine verglichen mit dem Material des Grundkörpers verschiedene Materialzusammensetzung aufweisen. Diese sogenannten (Röntgen-)Marker oder Funktionselemente enthalten insbesondere ein Material, das die Röntgenstrahlen und/oder andere elektromagnetische Strahlen stärker absorbiert (im Folgenden als röntgenopakes oder radioopakes Material bezeichnet) als das Material des Grundkörpers.

In der Druckschrift DE 10 2006 038 238 A1 wird ein Röntgenmarker für medizinische Implantate aus einem biokorrodierbaren metallischem Werkstoff und ein medizinisches Implantat mit einem derartigen Röntgenmarker beschrieben, wobei der Röntgenmarker ein Borid oder Carbid der Elemente Tantal oder Wolfram ist. Ein Marker mit einer derartigen Zusammensetzung weist jedoch nur eine geringe Röntgenabsorption auf, da die mittlere Ordnungszahl und die Dichte dieser Materialien gering sind.

Die Druckschrift US 2009/0204203 A1 offenbart ein Implantat in Form eines bioabsorbierbaren Stents mit einem oder mehreren radioopaken Markern. Eine Aufnahmeeinrichtung (marker support) an dem Körper des Stents, in der der Marker befestigt ist, wird vor dem Anbringen des Markers passiviert oder oxidiert. Hierdurch wird die Korrosion der Aufnahmeeinrichtung verlangsamt, so dass eine verbesserte Endothelialisierung erreicht wird.

Ferner wird in der Druckschrift DE 10 2008 054 845 A1 eine mechanische Verankerung von Tantal- oder Wolfram-Markern an Magnesium-Stents und eine anschließende plasmachemische Behandlung dieses Verbundes vorgeschlagen. Das dort angegebene Verfahren hat jedoch den Nachteil, dass die mechanische Verankerung von Markern, beispielsweise mittels Nieten, Laser- oder Elektronenstrahlverschweißen technologisch sehr anspruchsvoll ist und extremer feinmotorischer Fähigkeiten und/oder aufwendiger und somit teurer Handhabungstechnik bedarf. Ferner führt der metallische Kontakt von Tantal oder Wolf ram zu Magnesium, auch wenn dieser zunächst von außen passiviert ist, nach der Degradation der Passivierungsschicht zu einer dann beschleunigten Degradation, da die Materialien des Röntgenmarkers mit dem Material des Grundkörpers ein Lokalelement bilden.

EP-A-2184038 beschreibt Endoprothesen, insbesondere Stents, mit aus Magnesium bzw. Magnesiumlegierungen aufgebautem metallischem Grundkörper und einem an diesem befestigten radio- bzw. röntgenopaken Funktionselement enthaltend Metalle wie Wolfram, Tantal, Platin und/oder Gold, wobei das Funktionselement mindestens im Kontaktbereich zum Grundkörper durch eine Barriere-Schicht, z.B. Titandioxid elektrisch isoliert ist.

Wie oben bereits geschildert wurde, ergibt sich bei Implantaten, deren Grundkörper aus einem metallischen Material besteht, bei der Anordnung von metallischen Funktionselementen an dem Grundkörper das Problem, dass an dem Kontaktbereich zwischen dem Material des Grundkörpers und dem Material des Funktionselements eine Kontaktkorrosion auftritt. Diese führt zu beschleunigter Degradation bzw. zur Abtrennung des Funktionselements von dem Grundkörper, so dass entweder das Implantat nicht mehr in der Lage ist, seine Funktion zu erfüllen, oder nicht mehr aufgefunden werden kann. Für das geschilderte Problem enthalten die oben beschriebenen Vorrichtungen aus dem Stand der Technik keine oder nur unzureichende Lösungen.

Die Aufgabe der vorliegenden Erfindung besteht somit darin, ein Implantat zu schaffen, bei dem eine erhöhte Degradation im Bereich des Funktionselements vermieden und gleichzeitig eine hohe Verbundfestigkeit erzielt wird. Die Aufgabe besteht ferner darin, ein kostengünstiges Verfahren zur Herstellung eines derartigen Implantats anzugeben.

Die obige Aufgabe wird durch ein Implantat gelöst, bei dem das mindestens eine Funktionselement mindestens im Bereich seiner Oberfläche, an der es mit dem Grundkörper verbunden ist, eine erste Schicht aufweist, welche überwiegend mindestens ein Metalloxid enthält.

Ein derartiges erfindungsgemäßes Implantat hat den Vorteil, dass der metallische Kontakt zwischen dem Material des Funktionselements und dem Material des Grundkörpers durch die auf der Oberfläche des Funktionselements fest angeordnete erste Schicht mit dem mindestens einen Metalloxid von vornherein unterbunden wird. Hierdurch wird auch in einem fortgeschrittenen Stadium der Degradation, bei dem diese an der Grenzfläche zwischen Funktionselement und Grundkörper angelangt ist, eine Lokalelementbildung zwischen Grundkörper und Funktionselement unterbunden. Die erste Schicht mit einem Metalloxid hat auch den Vorteil, dass diese aufgrund ihrer Porosität eine Verbindung des Funktionselements mit dem Grundkörper erleichtert. Wie unten noch näher erläutert wird, besteht ein weiterer Vorteil der erfindungsgemäßen Lösung darin, dass die erste Schicht vor der Befestigung des Funktionselements an dem Grundkörper mittels einer Temperbehandlung oder einer elektrisch induzierten Glühbehandlung, erzeugt wird. Hierdurch wird vermieden, dass die Umgebung des Funktionselements an dem Grundkörper des Implantats thermisch belastet werden muss, was insbesondere hinsichtlich des tiefen Schmelzpunkts bzw. Schmelzbereichs der vorzugsweise verwendeten Magnesium-Legierungen (etwa 600°C bis 650°C) von Vorteil ist. Bei einer Temperbehandlung wird das Funktionselement über einen bestimmten Zeitraum in einem Ofen oder einem anderen beheizbaren Raum mit einer vorgegebenen Temperatur ausgelagert. Bei der elektrisch induzierten Glühbehandlung wird das Funktionselement in einen elektrischen Stromkreis eingebunden und aufgrund des bei einer vorgegebenen Gleichspannung fließenden Stroms und des eigenen Widerstands über einen festgelegten Zeitraum geglüht.

Alternativ wird die erste Schicht mittels einer plasmachemischen Behandlung des Funktionselements, bevorzugt mittels einer CORR-Behandlung, erzeugt. Bei der CORR-Behandlung werden höhere Endspannungen (bevorzugt größer als 400 V) und höhere Stromdichten (bevorzugt größer als 25 mA/cm²) verwendet als bei der herkömmlichen plasmachemischen Behandlung.

In einem bevorzugten Ausführungsbeispiel weist der Grundkörper mindestens ein zumindest weitestgehend biodegradierbares metallisches Material als Hauptbestandteil, vorzugsweise Magnesium oder eine Magnesiumlegierung, auf. Diese Materialien degradieren in einem Zeitfenster, welches für viele Anwendungen gewünscht wird.

In einer Weiterbildung der Erfindung weist das mindestens eine Funktionselement ein Metall oder eine Metalllegierung enthaltend mindestens ein Metall der Gruppe mit den Elementen Wolfram, Tantal und Titan sowie in seiner ersten Schicht mindestens ein Oxid der Gruppe mit den Verbindungen Wolframoxide, Tantaloxide und Titanoxide auf. Die angegebenen Materialen des Funktionselements besitzen gute Röntgenabsorptionseigenschaften und lassen sich durch die Oberflächenoxidation sehr einfach mit einer schon bei kleinen Schichtdicken dichten passivierenden und isolierenden (d.h. elektrisch nicht leitenden) Oxidschicht (erste Schicht) versehen. Sie erlauben hierdurch eine Gestaltung eines Funktionselements mit minimaler Größe.

Eine noch bessere Röntgensichtbarkeit wird erreicht, wenn das mindestens eine Funktionselement einen Mantel umfassend ein Metall oder eine Metalllegierung enthaltend mindestens ein Metall der Gruppe mit den Elementen Wolfram, Tantal und Titan sowie einen Kern umfassend vorzugsweise ein Metall oder eine Metalllegierung enthaltend mindestens ein Metall der Gruppe mit den Elementen Platin und Gold aufweist.

Hierbei muss der Mantel des Funktionselements den Kern nicht vollständig umschließen. Es ist lediglich notwendig, dass der Mantel den Kern mindestens in dem Bereich umschließt, in dem das Funktionselement an dem Grundkörper befestigt ist. Hierdurch wird sichergestellt, dass die Materialien Platin und Gold bzw. deren Legierungen nicht mit dem Material des Grundkörpers in Kontakt kommen und so ein Lokalelement ausbilden, dass im Falle von Gold oder Platin eine noch stärkere Degradation des Grundkörpers bewirken würde.

In einem weiteren bevorzugten Ausführungsbeispiel beträgt die Dicke der ersten Schicht 1 µm bis 20 µm, vorzugsweise 1 µm bis 10 µm. Durch die angegebene Dicke der ersten Schicht, die Metalloxide enthält, wird sichergestellt, dass eine ausreichende elektrische Isolierung des Funktionselements von dem Grundkörper erfolgt.

In einem weiteren bevorzugten Ausführungsbeispiel ist das mindestens eine Funktionselement mit einem Klebstoff, vorzugsweise mit einem polymerbasierten Klebstoff, an dem Grundkörper befestigt. Es wurde nämlich erkannt, dass insbesondere durch eine stoffschlüssige Verbindung eine einfache und mechanisch den filigranen Grundkörper nicht belastende Verbindung erzielt wird. Zusätzlich kann durch Anpassung der Form des Funktionselements und der Form der Aufnahme (Öffnung) am Grundkörper des Implantats, in welche das Funktionselement eingebracht wird, ein Formschluss realisiert werden.

Es wurde erkannt, dass herkömmliche Technologien für die Befestigung von Röntgenmarkern an einem Grundkörper, wie zum Beispiel Nieten, Crimpen, Laser- und Elekronenstrahlschweißen nicht ohne Weiteres für die Werkstoffe Magnesium oder Magnesiumlegierungen verwendet werden können. Magnesium oder Magnesiumlegierungen weisen gegenüber anderen, für Implantate verwendeten Materialien wie Nitinol einen geringeren Elastizitätsmodul und eine geringere Streckgrenze auf, die bei der Befestigung des Funktionselements zu einer zu großen thermischen und/oder mechanischen Beanspruchung des Grundkörpers in dem Bereich, in dem das Funktionselement angeordnet wird, und somit zu einer frühen und unerwünschten plastischen Verformung führen würde.

Als vorteilhafter polymerbasierter Klebstoff kann beispielsweise Polyurethan oder ein degradierbares Polymer (z. B. PLLA L210, PLLA L214) verwendet werden. Diese Klebstoffe sind besonders bioverträglich und sind außerdem gut elektrisch isolierend.

Die obige Aufgabe wird ferner durch ein Verfahren zur Herstellung eines derartigen Implantats gelöst, mit dem folgenden Schritt:
Befestigen mindestens eines Funktionselements an dem Grundkörper, das zumindest in einem Teil seines Volumens eine verglichen mit dem Material des Grundkörpers verschiedenen Materialzusammensetzung aufweist, die vorzugsweise radioopakes und/oder röntgenopakes Material umfasst, wobei das mindestens eine Funktionselement mindestens im Bereich seiner Oberfläche, an der es mit dem Grundkörper verbunden wird, eine erste Schicht aufweist, welche überwiegend mindestens ein Metalloxid enthält und wobei die erste Schicht vor der Befestigung des Funktionselements an dem Grundkörper mittels einer Temperbehandlung oder einer elektrisch induzierten Glühbehandlung oder mittels einer plasmachemischen Behandlung des Funktionselementes erzeugt wurde.

Das erfindungsgemäße Verfahren stellt ein kostengünstiges Verfahren zur Herstellung eines Implantats mit obigen Vorteilen dar.

Wie oben bereits erläutert wurde, ist es von Vorteil, wenn das mindestens eine Funktionselement durch Kleben, vorzugsweise mittels eines polymerbasierten Klebstoffs, an dem Grundkörper befestigt wird.

In einem besonders bevorzugten Ausführungsbeispiel wird das Funktionselement von einem draht- oder stabförmigen Halbzeug abgetrennt, wobei das Halbzeug
- vorzugsweise ein Metall oder eine Metalllegierung enthaltend mindestens ein Metall der Gruppe mit den Elementen Wolfram, Tantal und Titan oder
- vorzugweise einen Mantel umfassend ein Metall oder ein Metalllegierung enthaltend mindestens ein Metall der Gruppe mit den Elementen Wolfram, Tantal und Titan sowie einen Kern umfassend vorzugsweise ein Metall oder eine Metalllegierung enthaltend mindestens ein Metall der Gruppe mit den Elementen Platin und Gold
aufweist. Ein solches draht- oder stabförmiges Halbzeug kann vorzugsweise einen Durchmesser von 200 µm bis 400 µm besitzen.

Alternativ können die an dem Implantat zu befestigenden Funktionselemente auch als Ronden oder Pellets mit kreisförmigem, ovalem oder mehreckigem Querschnitt zur Verfügung gestellt werden. Diese sind vorzugsweise vor der Anordnung an dem Grundkörper mit der ersten Schicht versehen. Sie können beispielsweise mittels Kleben in einer entsprechenden Öffnung oder Ausnehmung am Grundkörper des Implantats befestigt werden.

Aus einem draht- oder stabförmigen Halbzeug können, nachdem durch eine Temperbehandlung die erste Schicht auf der Oberfläche des Halbzeugs erzeugt wurde, sehr kostengünstig durch Abtrennen einzelner Abschnitte von dem Halbzeug einzelne Funktionselemente erhalten werden. Diese können dann in entsprechend an dem Grundkörper geformte Aufnahmeeinrichtung, die beispielsweise eine zylindrische Ausnehmung oder Öffnung aufweist, verbracht und in diese eingeklebt werden. Hierzu wird die entsprechende Aufnahmeeinrichtung (im Folgenden auch Eyelet genannt) beispielsweise mit einem Übermaß von ca. 20 µm gefertigt. Vor dem Einkleben des Funktionselements in das Eyelet wird das Funktionselement in eine Polymerlösung getaucht und das Funktionselement dann in dem Eyelet angeordnet. Das Funktionselement kann sowohl vor der Abtrennung von dem Halbzeug als auch danach in dem Eyelet befestigt werden. Der Klebstoff wird dann beispielsweise durch eine Temperbehandlung, z.B. mittels IR-Strahlung, vernetzt und somit ausgehärtet.

Das draht- oder stabförmige Halbzeug kann beispielsweise einen kreisförmigen, ovalen oder mehreckigen (z.B. dreieckigen, quadratischen oder fünfeckigen) Querschnitt aufweisen. Es ist von Vorteil, wenn die ggf. auch durchgehende Ausnehmung oder Öffnung des Eyelets eine Querschnittsform aufweist, welche der Querschnittsform des Halbzeugs oder des Funktionselements angepasst ist, um einen zusätzlichen Formschluss zu realisieren.

Insbesondere bevorzugt ist, wenn das draht- oder stabförmige Halbzeug vorzugsweise vor der Befestigung an dem Grundkörper und/oder vorzugsweise vor der Erzeugung der ersten Schicht mit mindestens einer Sollbruchstelle versehen wird. Diese Sollbruchstelle ermöglicht es, einzelne Funktionselemente mit geringer Kraft von dem Halbzeug zu trennen. Vorzugsweise beträgt der Durchmesser der Sollbruchstelle höchstens ca. 30% des Durchmessers des Halbzeugs. Die Sollbruchstelle wird vorzugsweise mittels eines spanenden Verfahrens, beispielsweise mittels Drehen, hergestellt.

Durch einen vor der Befestigung des Funktionselements an dem Grundkörper durchgeführten Prozessschritt, welcher zur Ausbildung der ersten Schicht durchgeführt wird, beispielsweise eine Temperbehandlung, eine elektrisch induzierte Glühbehandlung oder eine plasmachemische Behandlung des Halbzeugs, kann in einem weiteren Ausführungsbeispiel die Sollbruchstelle, die zunächst vorzugsweise mechanisch, z.B. mittels eines Trenn- oder Schneidewerkzeugs, in das Halbzeug eingebracht wird, weiter vertieft, beispielsweise auf einen Restquerschnitt des Halbzeugs an der Sollbruchstelle von 20 µm bis 70 µm reduziert werden. Hierdurch ist nur noch eine sehr geringe Kraft nötig, um den Abschnitt des Halbzeugs, der aktuell das Funktionselement ausbilden soll, von dem restlichen Teil des Halbzeugs zu trennen (z.B. mittels einer Zange).

Die Temperbehandlung zur Aushärtung des Klebstoffs kann ggf. vor oder nach dem Abtrennen des Funktionselements von dem Halbzeug durchgeführt werden.

Es ist weiter von Vorteil, wenn die Befestigung des Funktionselements an dem Grundkörper nach einem Laserschneiden des Grundkörpers erfolgt. Die raue Laserschnittkante erhöht die Adhäsion des Klebstoffs oder anderer Befestigungsmittel an dem Grundkörper.

Anschließend können gegebenenfalls ein oder mehrere weitere Prozessschritte zur Herstellung der endgültigen Form des Implantats durchgeführt werden, wie manuelles Entgraten, Reibahlen, Verschleifen der Innen- und Außenoberfläche mit abrasiv wirkenden Domen (innen) und/oder Hülsen (außen). Hierdurch werden auch überstehende Abschnitte oder Kanten des Funktionselements entfernt.

Danach kann das Implantat einer Elektropolitur und/oder einer plasmachemischen Behandlung unterzogen werden. Die der Elektropolitur und/oder plasmachemischen Behandlung vorgelagerten, im vorangegangenen Absatz erwähnten weiteren Prozessschritte können auch entfallen.

Hinsichtlich der plasmachemischen Behandlung und/oder der Elektropolitur besteht ein weiterer Vorteil des erfindungsgemäßen Implantats darin, dass das Funktionselement durch die erste Schicht elektrisch von dem Grundkörper entkoppelt ist, so dass durch das plasmachemische Verfahren und/oder die Elektropolitur lediglich an der Oberfläche des Grundkörpers eine Schicht mit Oxiden und ggf. auch Phosphaten hergestellt wird, welche in Bezug auf ihre Zusammensetzung und Dicke entsprechend der gewünschten Degradationsdauer eingestellt werden kann.

Bei der plasmachemischen Behandlung (Beschichtung) wird an den Grundkörper eine gepulste Spannung angelegt, deren Amplitude in einem Teil des Behandlungszeitraums eine für das Material des Grundköpers charakteristische Badspannung übersteigt und vorzugsweise im Verlauf der Behandlung ansteigt.

In einem bevorzugten Ausführungsbeispiel beträgt die Stromdichte bei der plasmachemischen Behandlung mindestens etwa 4 mA/cm².

Zur plasmachemischen Behandlung erfolgt eine anodische Kontaktierung des Grundkörpers. Der Kontaktierungswerkstoff besteht dabei aus einem Aluminium- oder Titan-Draht. Nun wird das Implantat in eine wässrige, vorzugsweise phosphathaltige Lösung eingetaucht. Nach Anlegen einer gepulsten, stetig steigenden Badspannung, die sich durch lange Pulspausen auszeichnet, kommt es zur Oxidation des Werkstoffs des Grundkörpers. Die plasmachemischen Effekte lassen eine Mischphase aus Oxiden und gegebenenfalls Phosphaten des Materials des Grundkörpers entstehen. Es ist hierbei von Vorteil, wenn die jeweiligen Oxidschichten langsam wachsen, da andernfalls das Oxid mit der höchsten Bildungsenthalpie keine kohärente Schicht ausbilden kann. Das langsame Wachstum der Oxidschicht wird erfindungsgemäß durch lange Pausen zwischen den Spannungspulsen erreicht. Diese können bis zu 500 Millisekunden lang sein.

Die plasmachemische Behandlung erzeugt auf dem Metall des Grundkörpers eine verfahrensbedingt poröse Struktur, die eine nachträgliche Versiegelung des Bauteils, z.B. mit einer polymeren Deckschicht, durch eine bessere Haftung vereinfacht oder die als Carrier oder Träger (bspw. für eine pharmazeutisch aktive Substanz oder andere funktionelle Stoffe) genutzt werden kann.

Hierbei wird unter einer "pharmazeutisch aktiven Substanz" (oder therapeutisch aktive oder wirksame Substanz) ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom Menschen oder tierischen Körper erzeugte Wirkstoffe, wie Insulin, sowie zur Beseitigung oder Unschädlichmachen von Krankheitserregern, Tumoren, Krebszellen oder Körperfremdstoffen Einsatz findet.

Vorzugsweise wird der Grundkörper nach der plasmachemischen Behandlung in einem Lösungsmittel, vorzugsweise mittels destilliertem H₂O gespült und anschließend vorzugsweise bei einer Temperatur von mindestens 80°C, besonders bevorzugt mindestens etwa 100°C getrocknet, wobei das Trocknen vorzugsweise in einem Umluftofen durchgeführt wird.

Es ist weiter von Vorteil, wenn in der wässrigen Lösung ein Puffer, vorzugsweise Kaliumdihydrogenphosphat und/oder Natriumdihydrogenphosphat enthalten ist. Alternativ oder zusätzlich kann in der wässrigen Lösung auch Calciumdihydrogenphosphat als Puffer enthalten sein, dessen geringe Wasserlöslichkeit durch die Zugabe von Komplexbildnern wie Ethylendiamin erhöht werden kann. Hierdurch werden insbesondere Phosphate in der plasmachemisch erzeugten Schicht auf dem Grundkörper erzeugt.

Als weitere, abschließende Oberflächenbehandlung kann das Implantat in einem weiteren Ausführungsbeispiel vorzugsweise nach der plasmachemischen Behandlung oder der Elektropolitur mit einer zweiten Schicht aufweisend ein degradierbares Polymer versehen werden, vorzugsweise mittels Sprühen. Die plasmachemische Behandlung oder Elektropolitur kann auch entfallen. Eine solche zweite Schicht hat eine Schichtdicke von etwa 1 µm bis etwa 10 µm, vorzugsweise etwa 1 µm bis etwa 5 µm. Als degradierbares Polymer kann beispielsweise PLLA L210 eingesetzt werden.

Die Erfindung wird nachfolgend anhand von in Figuren dargestellten Ausführungsbeispielen näher erläutert. Dabei bilden alle beschriebenen und/oder bildlich dargestellten Merkmale den Gegenstand der Erfindung, auch unabhängig von ihrer Zusammenfassung in den Ansprüchen oder deren Rückbezügen.

Es zeigen schematisch:
- Fig. 1: einen Querschnitt durch ein an einem Grundkörper befestigtes Funktionselement eines ersten Ausführungsbeispiels eines erfindungsgemäßen Implantats,
- Fig. 2: einen Querschnitt durch ein an einem Grundkörper befestigtes Funktionselement eines zweiten Ausführungsbeispiels eines erfindungsgemäßen Implantats,
- Fig. 3: einen Querschliff eines Wolfram-Stabes mit einer Oxidschicht und
- Fig. 4: einen Wolfram-Stab mit einer Sollbruchstelle in einer Ansicht von der Seite.

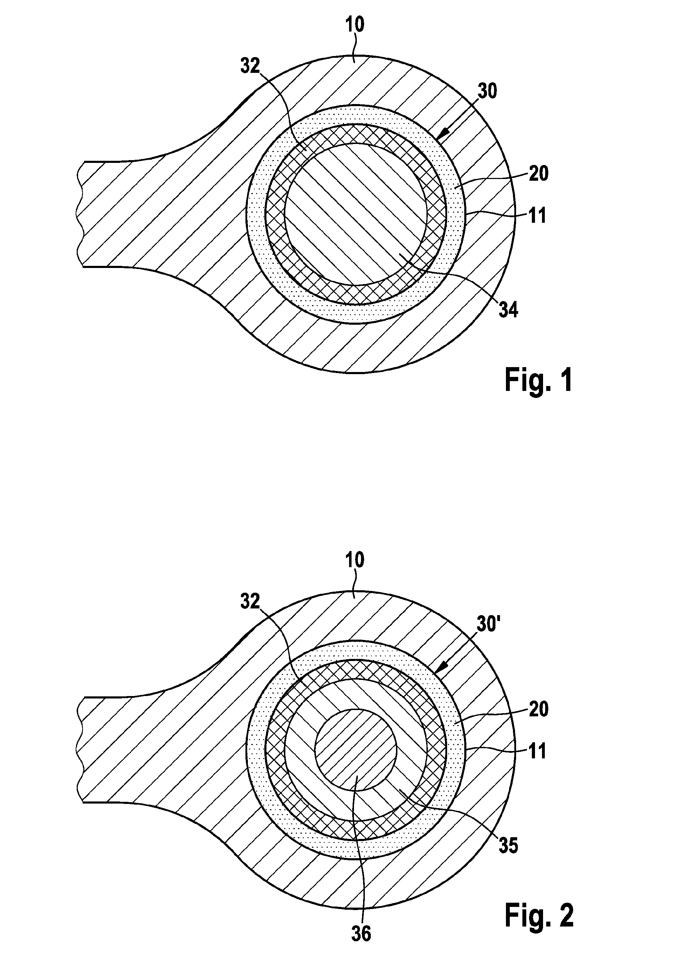

In Fig. 1 ist ein Abschnitt eines Grundkörpers eines erfindungsgemäßen Implantats in Form eines medizinischen Stents bestehend aus der Magnesiumlegierung WE43 mit einem Funktionselement 30 dargestellt. Das Bild zeigt ein beispielsweise am distalen oder proximalen Ende des Grundkörpers angeordnetes Eyelet 10. Vorzugsweise sind am distalen und/oder proximalen Ende des Grundkörpers des Implantats jeweils drei um 120° versetzte solche Eyelets als Bestandteile des Grundkörpers am hohlzylinderförmigen Gitter, beispielsweise an einer Strebe (Strut), ausgebildet.

In einer zylinderförmigen, durchgehenden Ausnehmung 11 des Eyelets 10, welche auch die innere Oberfläche des Eyelets 10 bildet, ist ein röntgenopakes Funktionselement 30 angeordnet, das mittels einer Kleberschicht 20 an dem Eyelet 10 befestigt ist. An seiner äußeren Oberfläche, mit der das Funktionselement (Röntgenmarker) 30 mit der inneren Oberfläche des Eyelets 10 verbunden ist, weist das Funktionselement 30 eine erste Schicht 32 auf, welche überwiegend aus Wolframoxiden besteht. Das im Wesentlichen zylinderförmige Funktionselement 30 besteht in einem inneren Bereich 34 unterhalb oder innerhalb der ersten Schicht 32 aus Wolfram. Vorzugsweise ist die erste Schicht 32 hohlzylinderförmig ausgebildet und umgibt den inneren, im Wesentlichen zylinderförmigen Bereich 34 lediglich an seiner Mantelfläche.

In dem in Figur 2 dargestellten Ausführungsbeispiel weist das Funktionselement 30' unterhalb der ersten Schicht 32 bestehend überwiegend aus Wolframoxiden eine im Wesentlichen hohlzylinderförmige Wolframschicht 35 auf, welche als Mantel einen beispielsweise aus Gold und/oder Platin oder einer Legierung mit diesen Elementen bestehenden, im Wesentlichen zylinderförmigen Kern 36 des Funktionselements 30' umgibt. Auch bei diesem Ausführungsbeispiel ist die erste Schicht 32 vorzugsweise hohlzylinderförmig ausgebildet und umgibt die hohlzylinderförmige Wolframschicht 35 lediglich im Bereich ihrer Mantelfläche. Das Funktionselement 30' ist aufgrund der höheren Röntgendichte des Kerns 36 gegenüber dem Material des inneren Bereichs 34 des Funktionselements 30 des ersten Ausführungsbeispiels noch besser im Röntgenbild sichtbar.

Ein solches Funktionselement 30' kann beispielsweise einen Außendurchmesser von 400 µm und eine etwa 5 µm dicke erste Schicht 32 aufweisen. Der Wolfram-Mantel 35, der alternativ auch aus Titan bestehen kann, besitzt beispielsweise einen Außendurchmesser von 390 µm Der Außendurchmesser des zylinderförmigen Kerns 36 aus Gold und/oder Platin oder einer Legierung dieser Elemente kann z.B. 350 µm betragen.

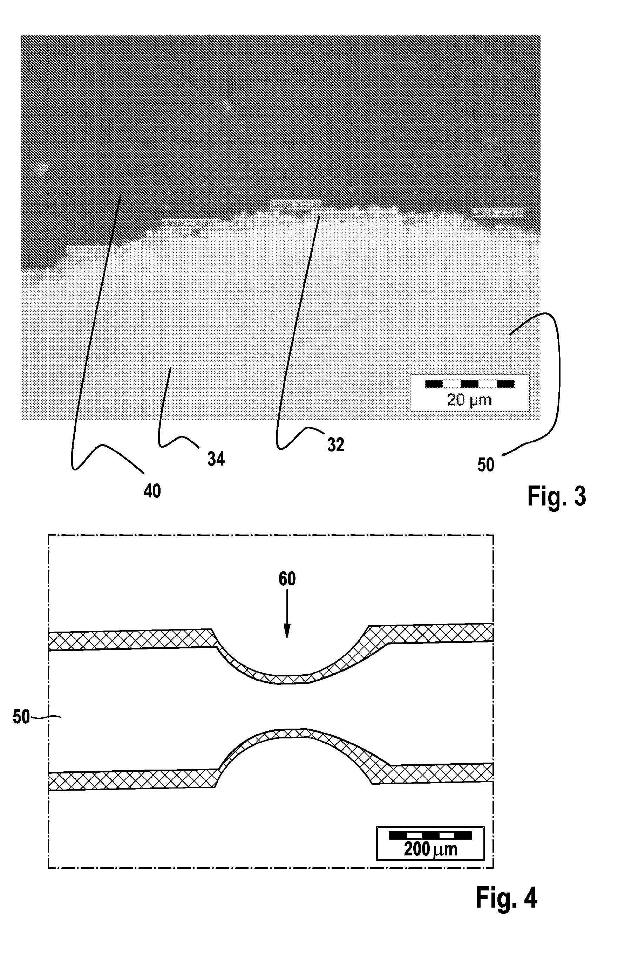

Das Funktionselement 30 des in Figur 1 dargestellten ersten Ausführungsbeispiels wird in einem ersten Ausführungsbeispiel des erfindungsgemäßen Verfahrens aus einem drahtförmigen Halbzeug in Form einer Stange 50 mit einem im Wesentlichen kreisförmigen Querschnitt hergestellt (Figur 4). Die Stange 50 besteht aus Wolfram und weist einen Durchmesser von etwa 400 µm auf. Eine solche Stange 50 wird in Abständen von beispielsweise 120 µm mit Sollbruchstellen 60 versehen, welche jeweils einen Durchmesser von etwa 30 µm bis etwa 80 µm aufweisen. Die Sollbruchstellen 60 werden mittels spanender Verfahren wie Drehen (durch Einstechen mit Drehmeißel oder durch Einschleifen mittels einer spitzen, schnell drehenden Schleifscheibe, die beispielsweise eine Dicke von ca. 20 µm aufweist) erzeugt. Jeder Abschnitt der Stange 50, welcher über Sollbruchstellen 60 von einem weiteren getrennt ist, soll nach Abschluss des Herstellungsverfahrens ein Funktionselement 30 ausbilden. Ein Abschnitt einer solchen Stange 50 aus Wolfram mit einer Sollbruchstelle 60 ist in Figur 4 dargestellt. Die hellen Bereiche in der Mitte der Stange 50 und die grauen Randbereiche stellen Lichtreflexe dar.

Eine solche mit Sollbruchstellen 60 versehene Stange 50 wird nun an eine Spannungsquelle angeschlossen. Dann wird bei einer Gleichspannung von 3 V eine Stromstärke von 15 A über einen Zeitraum von 5 Sekunden angelegt. Hierdurch wird die Stange 50 erwärmt, wobei die Erwärmung durch Glühfarben (hier hellgelb) sichtbar ist. Dadurch entsteht eine dichte Oxidschicht (erste Schicht 32) mit einer Dicke von etwa 2 µm bis 3 µm auf der Oberfläche der Stange 50, welche überwiegend Wolframoxide aufweist. Ein Querschliff einer so behandelten Stange 50 ist in Fig. 3 dargestellt. Zur genaueren Untersuchung wurde die mit der ersten Schicht 32 versehene Stange 50 in ein Einbettmittel 40 eingebettet und ein Querschliff erzeugt. Auf der fotografischen Aufnahme ist zu erkennen, dass die erste Schicht 32 eine Dicke von wenigen µm aufweist. Die gemessenen Dicken liegen zwischen 2,2 µm und 3,2 µm. Alternativ können Stangen mit Wolfram auch in einem Ofen an Luft bei Temperaturen von ca. 600°C über 2h geglüht werden. Dabei bilden sich ähnlich dicke Oxidschichten.

Durch die oben beschriebene Passivierung der Stange 50 durch Ausbildung der ersten Schicht 32 werden auch die metallischen Restquerschnitte der Sollbruchstellen 60 weiter verringert bis sie einen Durchmesser von etwa 20 µm bis etwa 70 µm aufweisen. Der nun vorhandene geringe metallische Restquerschnitt der Sollbruchstellen 60 weist eine für den Prozess der Montage in das Eyelet noch ausreichende mechanische Festigkeit, insbesondere eine ausreichende Biegefestigkeit, auf. Bei der beim anschließenden Trennen mittels Zange vorliegenden Scherbeanspruchung können die Sollbruchstellen 60 jedoch ohne gro-ße mechanische Kräfte durchtrennt werden.

Die mit der ersten Schicht 32 versehene Stange 50 wird anschließend in ein mit einem Lösungsmittel versetztes Polymer aus Polyurethan (PU) getaucht und nach einigen Sekunden Verweilzeit wieder aus dem Behälter gezogen. Das an der Oberfläche der Stange 50 haftende Polymer weist dann eine Schichtdicke von etwa 20 µm auf. Die Stange wird dann in die Öffnung 11 des Eyelets 10, welches beispielsweise einen Innendurchmesser von etwa 450 µm besitzt, verbracht. Hierbei ist der Stent auf einem Dorn fixiert und in der Öffnung 11 des Eylets 10 ist genau ein Abschnitt der Stange, welches dann das entsprechende Funktionselement bilden soll, angeordnet. Anschließend wird der PU-Klebstoff mittels IR-Strahlung vernetzt. Hierdurch härtet dieser aus und der das Funktionselement 30 ausbildende Abschnitt des Stabes 50 kann an der Sollbruchstelle 60 mit einer Zange von dem restlichen Teil der Stange 50 abgetrennt werden. Hierbei entsteht nur eine minimale Biegebelastung, so dass die über dem Klebstoff 20 wirkende Verbindung zwischen Funktionselement 30 und Grundkörper (Eyelet 10) und der filigrane Grundkörper mechanisch nur sehr wenig beansprucht werden.

Anschließend wird der mit den Funktionselementen versehene Stent entgratet, mittels Reibahlen behandelt und/oder gegebenenfalls die Innen- und Außenseiten des Stents mechanisch geschliffen. Hierdurch werden auch überstehende Bereiche des Funktionselements 30 mechanisch entfernt.

Anschließend wird der so behandelte Stent in einer wässrigen phosphorsäurehaltigen Lösung elektropoliert. Die Lösung enthält beispielsweise:
20 Volumenteile Wasser
30 Volumenteile 85%-ige Phosphorsäure
50 Volumenteile Ethanol

Die Badspannung beträgt ca. 6V (Gleichspannung).

Durch das Elektropolieren wird das Funktionselement 30 nicht verändert, da dieses durch die erste Schicht 32 und den Klebstoff 20 gegenüber dem Grundkörper (Eyelet 10) elektrisch isoliert ist.

In einem zweiten Ausführungsbeispiel wird das oben beschriebene Herstellungsverfahren mit einer Stange aus Tantal (statt Wolfram) durchgeführt. Abweichend von den oben angegebenen Parametern für die Wolfram-Stange wird bei der Tantal-Stange zur Erzeugung der ersten Schicht 32 (Oxidschicht aufweisend überwiegend Tantaloxide) eine Gleichspannung von 3 V und einer Stromstärke von 10 A über einen Zeitraum von 5 Sekunden angelegt.

In einem dritten Ausführungsbeispiel des erfindungsgemäßen Verfahrens kann als Halbzeug für die Herstellung des Funktionselements ein Stab verwendet werden, welcher einen Hohlzylinder-Mantel aus Titan mit einem Außendurchmesser von 400 µm und einen Kern bestehend aus Gold mit einem Außendurchmesser von 350 µm aufweist.

Im Gegensatz zum ersten Ausführungsbeispiel wird zudem die auf dem Titan-Mantel angeordnete erste Schicht (Oxidschicht) mittels plasmachemischer Behandlung (im Folgenden auch CORR-Behandlung genannt) erzeugt, wobei die erste Schicht überwiegend Titanoxide enthält. Durch die CORR-Behandlung entsteht eine ca. 5 µm dicke dielektrische mikroporöse Oberflächenmorphologie, welche eine sehr gute Benetzung mit Klebstoff und somit eine gute stoffschlüssige Verbindung zwischen dem Funktionselement und dem Grundkörper (Eyelet) bewirkt. Die CORR-Behandlung für den Titan ummantelten Stab erfolgt in der gleichen Elektrolytzusammensetzung wie die im fünften Ausführungsbeispiel beschriebene plasmachemische Behandlung des Grundkörpers. Die Endspannung beträgt bei der CORR-Behandlung jedoch 480 V. Die Pulslänge der gepulsten Gleichspannung (pulse on) beträgt 5 µs, die Pulspause (pulse off) beträgt 1000 µs. Die Stromdichte beträgt ca. 25 mA/cm². Das heißt, der mit Titan ummantelte Stab wird anodisch kontaktiert, in einer frei zu wählenden Tiefe in den Elektrolyt eingetaucht und mit einer von 0 V ansteigenden Badspannung bis zur Endspannung beaufschlagt. Nach ca. 2 Minuten Beschichtungszeit ist die Endspannung erreicht, die Stromzufuhr wird unterbrochen, anschließend der Stab aus dem Elektrolyt entnommen und an Warmluft getrocknet.

Das vierte Ausführungsbespiel entspricht dem zweiten Ausführungsbeispiel des erfindungsgemäßen Verfahrens, wobei statt einem Polyurethan nun ein biodegradierbares Polymer, beispielsweise PLLA L210, als Klebstoff verwendet wird.

Das fünfte Ausführungsbeispiel entspricht dem vierten Ausführungsbeispiel des erfindungsgemäßen Verfahrens, wobei anstelle der Elektropolitur anschließend eine plasmachemische Behandlung des Grundkörpers des Implantats erfolgt. Hierfür wird der Grundkörper anodisch kontaktiert und in einen wässrigen Elektrolyt folgender Zusammensetzung (bezogen auf 1 Liter H₂O):
80 g KH₂P0₄
45 g Na₂C0₃
65 ml ED (99%)
5 g bis 10 g NaOH
eingetaucht und plasmachemisch oxidiert.

Bei Badendspannungen von 200 bis 500 V und unter Verwendung von gepulsten Strömen mit einem Tastverhältnis von 5 µs on und 1000 µs off (im Extremfall bis zu 5000 Mikrosekunden off) und einer Stromdichte von 1 mA/cm² entsteht an der Oberfläche des Grundkörpers eine Schicht aus Oxiden, Mischoxiden, Phosphaten und Mischphosphaten sowie Spinelle des jeweiligen metallischen Grundwerkstoffs. Die einzustellende Badendspannung ist dabei abhängig vom Material des Grundkörpers. So wird beispielsweise eine Schichtdicke von 3 µm auf der Magnesiumlegierungsoberfläche bei einer Badendspannung von 260 V erzielt. Nach Erreichen dieser Endspannung fällt die Stromdichte bis auf die Hälfte des ursprünglich eingestellten Wertes ab. Danach wird die Stromzufuhr beendet, der Stent dem Bad entnommen und intensiv unter fließendem destilliertem Wasser gespült und an Warmluft bei ca. 40°C getrocknet. Abschließend wird der Stent sorgfältig vom Kontaktierungsmaterial getrennt und trocken bzw. unter Inertatmosphäre bis zu seiner Weiterverarbeitung gelagert.

Anschließend kann beispielsweise mittels Sprühen noch eine zweite Schicht aus einem biodegradierbaren Polymer, beispielsweise PLLA L210 oder PLLA L214 aufgebracht werden.

Durch das erfindungsgemäße Herstellungsverfahren können Implantate produziert werden, welche ihre mechanischen Eigenschaften während der und durch die Befestigung des Funktionselements nicht ändern. Ferner wird eine degradationsbeschleunigende Lokalelementbildung durch das Funktionselement mit der Oxidschicht wirkungsvoll verhindert. Die Verwendung eines Klebstoffs zur Verbindung von Kontaktelementen und Grundkörper ist vorteilhaft, da sich durch die poröse Struktur der Oxidschicht eine hohe Adhäsion des Klebstoffs und somit eine gute Haftung ergibt. Hierdurch wird ein Herauslösen des Kontaktelements bei einer plastischen Verformung des Implantats, beispielsweise bei einer Dilatation eines Stents, verhindert. Das erfindungsgemäße Verfahren stellt zudem sicher, dass die Genauigkeit des Fertigungsprozesses erhöht wird und das System aufgrund der Mehrlagen-Anordnung eine hohe Sicherheitstoleranz gegen eine beschleunigte Degradation besitzt.

### Bezugszeichenliste

- 10: Eyelet
- 11: Öffnung
- 20: Klebstoffschicht
- 30, 30`: Funktionselement
- 32: erste Schicht
- 34: innerer Bereich
- 35: Mantel
- 36: Kern
- 40: Einbettmittel
- 50: Stange
- 60: Sollbruchstelle

## Patentansprüche

1. Implantat, insbesondere eine intraluminale Endoprothese, mit einem Grundkörper, der mindestens teilweise aus einem biodegradierbaren metallischen Material besteht, und mit mindestens einem an dem Grundkörper befestigten Funktionselement (30, 30'), das zumindest in einem Teil seines Volumens eine verglichen mit dem Material des Grundkörpers verschiedene Materialzusammensetzung aufweist, die vorzugsweise radioopakes und/oder röntgenopakes Material umfasst, **dadurch gekennzeichnet*,* dass** das mindestens eine Funktionselement (30, 30') mindestens im Bereich seiner Oberfläche, an der es mit dem Grundkörper verbunden ist, eine erste Schicht (32) aufweist, welche überwiegend mindestens ein Metalloxid enthält und wobei die erste Schicht vor der Befestigung des Funktionselements an dem Grundkörper mittels einer Temperbehandlung oder einer elektrisch induzierten Glühbehandlung oder mittels einer plasmachemischen Behandlung des Funktionselements erzeugt wurde

2. Implantat nach Anspruch 1, **dadurch gekennzeichnet, dass** der Grundkörper mindestens ein Magnesium oder eine Magnesiumlegierung als Hauptbestandteil, aufweist.

3. Implantat nach einem der vorhergehenden Ansprüche, **dadurch gekennzeichnet, dass** das mindestens eine Funktionselement (30, 30') ein Metall oder eine Metalllegierung enthaltend mindestens ein Metall der Gruppe mit den Elementen Wolfram, Tantal und Titan sowie in seiner ersten Schicht mindestens ein Oxid der Gruppe mit den Verbindungen Wolframoxide, Tantaloxide und Titanoxide aufweist.

4. Implantat nach einem der vorhergehenden Ansprüche, **dadurch gekennzeichnet, dass** das mindestens eine Funktionselement (30, 30') einen Mantel (35) umfassend ein Metall oder eine Metalllegierung enthaltend mindestens ein Metall der Gruppe mit den Elementen Wolfram, Tantal und Titan sowie einen Kern (36) umfassend vorzugsweise ein Metall oder eine Metalllegierung enthaltend mindestens ein Metall der Gruppe mit den Elementen Platin und Gold aufweist.

5. Implantat nach einem der vorhergehenden Ansprüche, **dadurch gekennzeichnet, dass** die Dicke der ersten Schicht (32) etwa 1 µm bis etwa 20 µm, vorzugsweise etwa 1 µm bis etwa 10 µm beträgt.

6. Implantat nach einem der vorhergehenden Ansprüche, **dadurch gekennzeichnet, dass** das mindestens eine Funktionselement (30, 30') mit einem Klebstoff (20), vorzugsweise mit einem polymerbasierten Klebstoff, an dem Grundkörper befestigt ist.

7. Verfahren zur Herstellung eines Implantats, insbesondere einer intraluminalen Endoprothese, mit einem Grundkörper, der zumindest teilweise aus einem biodegradierbaren metallischen Material besteht, mit dem folgenden Schritt:
Befestigen mindestens eines Funktionselements (30, 30') an dem Grundkörper, das zumindest in einem Teil seines Volumens eine verglichen mit dem Material des Grundkörpers verschiedene Materialzusammensetzung aufweist, die vorzugsweise radioopakes und/oder röntgenopakes Material umfasst, wobei das mindestens eine Funktionselement (30, 30') mindestens im Bereich seiner Oberfläche, an der es mit dem Grundkörper verbunden wird, eine erste Schicht (32) aufweist, welche überwiegend mindestens ein Metalloxid enthält, und wobei die erste Schicht vor der Befestigung des Funktionselements an dem Grundkörper mittels einer Temperbehandlung oder einer elektrisch induzierten Glühbehandlung oder mittels einer plasmachemischen Behandlung des Funktionselements erzeugt wurde.

8. Verfahren nach Anspruch 7, **dadurch gekennzeichnet, dass** das mindestens eine Funktionselement (30, 30') durch Kleben, vorzugsweise mittels eines polymerbasierten Klebstoffs, an dem Grundkörper befestigt wird.

9. Verfahren nach einem der Ansprüche 7 bis 8, **dadurch gekennzeichnet, dass** das Funktionselement von einem draht- oder stabförmigen Halbzeug (50) abgetrennt wird, wobei das Halbzeug (50) vorzugsweise einen Mantel umfassend ein Metall oder eine Metalllegierung enthaltend mindestens ein Metall der Gruppe enthaltend Wolfram, Tantal und Titan sowie einen Kern (36) umfassend vorzugsweise ein Metall oder eine Metalllegierung enthaltend mindestens ein Metall der Gruppe enthaltend Platin und Gold aufweist.

10. Verfahren nach Anspruch 9, **dadurch gekennzeichnet, dass** das draht- oder stabförmige Halbzeug (50) vorzugsweise vor der Befestigung an dem Grundkörper und/oder vorzugsweise vor der Erzeugung der ersten Schicht mit mindestens einer Sollbruchstelle (60) versehen wird.

11. Verfahren nach Anspruch 10, **dadurch gekennzeichnet, dass** die mindestens eine Sollbruchstelle (60) während eines an dem Halbzeug (50) durchgeführten Prozessschrittes, der vor der Befestigung des Funktionselements an dem Grundkörper zur Ausbildung der ersten Schicht (32) durchgeführt wird, vertieft wird.

12. Verfahren nach einem der Ansprüche 7 bis 11, **dadurch gekennzeichnet, dass** die Befestigung des Funktionselements (30, 30`) an dem Grundkörper nach einem Laserschneiden des Grundkörpers erfolgt.

13. Verfahren nach einem der Ansprüche 7 bis 12, **dadurch gekennzeichnet, dass** das Implantat nach dem Befestigen des Funktionselements (30, 30`) an dem Grundkörper einer plasmachemischen Behandlung und/oder einer Elektropolitur unterzogen wird.

14. Verfahren nach Anspruch 13, **dadurch gekennzeichnet, dass** die plasmachemische Behandlung dadurch erfolgt, dass an den Grundkörper eine gepulste Spannung angelegt wird, deren Amplitude in einem Teil des Behandlungszeitraums eine für das Material des Grundkörpers charakteristische Badspannung übersteigt und vorzugsweise im Verlauf der Behandlung ansteigt.

15. Verfahren nach einem der Ansprüche 7 bis 14, **dadurch gekennzeichnet, dass** das Implantat vorzugsweise nach der plasmachemischen Behandlung mit einer zweiten Schicht aufweisend ein degradierbares Polymer, vorzugsweise mittels Sprühen, versehen wird.

## Claims

1. An implant, in particular an intraluminal endoprosthesis, comprising a main body that at least partially consists of a biodegradable metal material, and comprising at least one functional element (30, 30') that is fastened to the main body and has a material composition in at least a portion of its volume that differs from the material of the main body, the material composition preferably including radiopaque and/or x-ray opaque material, **characterised in that** the at least one functional element (30, 30') includes a first layer (32) at least in the region of its surface where it is bonded to the main body, the first layer primarily containing at least one metal oxide, and wherein the first layer is created, using a thermal treatment or an electrically induced annealing treatment or using a plasma-chemical treatment of the functional element, before the functional element is fastened to the main body.

2. The implant according to Claim 1, **characterised in that** the main body contains at least one magnesium or a magnesium alloy as the main component.

3. The implant according to one of the preceding claims, **characterised in that** the at least one functional element (30, 30') contains a metal or a metal alloy that includes at least one metal of the group comprising the elements tungsten, tantalum, and titanium, and, in its first layer, contains at least one oxide of the group comprising the compounds tungsten oxides, tantalum oxides, and titanium oxides.

4. The implant according to one of the preceding claims, **characterised in that** the at least one functional element (30, 30') has a jacket (35) containing a metal or a metal alloy that includes at least one metal of the group comprising the elements tungsten, tantalum, and titanium, and a core (36) preferably comprising a metal or a metal alloy that includes at least one metal of the group comprising the elements platinum and gold.

5. The implant according to one of the preceding claims, **characterised in that** the thickness of the first layer (32) is approximately 1 µm to approximately 20 µm, preferably approximately 1 µm to approximately 10 µm.

6. The implant according to one of the preceding claims, **characterised in that** the at least one functional element (30, 30') is fastened to the main body using an adhesive (20), preferably a polymer-based adhesive.

7. A method for producing an implant, in particular an intraluminal endoprosthesis, comprising a main body that at least partially consists of a biodegradable metal material, said method having the following step:
fastening at least one functional element (30, 30') to the main body, which functional element has a material composition in at least a portion of its volume that differs from the material of the main body, the material composition preferably including radiopaque and/or x-ray opaque material, wherein the at least one functional element (30, 30') includes a first layer (32) at least in the region of its surface where it is bonded to the main body, the first layer primarily containing at least one metal oxide, and wherein the first layer is created, using a thermal treatment or an electrically induced annealing treatment or using a plasma-chemical treatment of the functional element, before the functional element is fastened to the main body.

8. The method according to Claim 7, **characterised in that** the at least one functional element (30, 30') is fastened to the main body by bonding, preferably using a polymer-based adhesive.

9. The method according to any one of Claims 7 to 8, **characterised in that** the functional element is cut from a wire- or bar-shaped semi-finished product (50), wherein the semi-finished product (50) preferably includes a jacket containing a metal or a metal alloy that includes at least one metal of the group that includes tungsten, tantalum, and titanium, and a core (36) that preferably contains a metal or a metal alloy that includes at least one metal of the group comprising platinum and gold.

10. The method according to Claim 9, **characterised in that** the wire- or bar-shaped semi-finished product (50) is preferably provided with at least one predetermined breaking point (60) before it is fastened to the main body, and/or preferably before the first layer is created.

11. The method according to Claim 10, **characterised in that** the at least one predetermined breaking point (60) is hollowed out during a process step that is carried out on the semi-finished product (50) to form the first layer (32) before the functional element is fastened to the main body.

12. The method according to one of Claims 7 to 11, **characterised in that** the functional element (30, 30') is fastened to the main body after a laser-beam cutting of the main body has been performed.

13. The method according to one of Claims 7 to 12, **characterised in that** the implant is subjected to a plasma-chemical treatment and/or electropolishing after the functional element (30, 30') is fastened to the main body.

14. The method according to Claim 13, **characterised in that** the plasma-chemical treatment is carried out by applying a pulsed voltage to the main body, the amplitude of which pulsed voltage exceeds, over a portion of the treatment period, a bath voltage that is characteristic for the material of the main body, said pulsed voltage optionally increasing over the course of the treatment.

15. The method according to one of Claims 7 to 14, **characterised in that** the implant is provided with a second layer containing a degradable polymer, preferably after the plasma-chemical treatment, and preferably by means of spraying.

## Revendications

1. Implant, notamment, endoprothèse intraluminale, avec un corps de base qui est constitué au moins partiellement d'un matériau métallique pouvant être dégradé biologiquement, et avec au moins un élément fonctionnel (30, 30'), fixé sur le corps de base, qui présente dans au moins une partie de son volume une composition d'un matériau différente du matériau du corps de base, qui comprend de préférence un matériau radio-opaque et/ou opaques aux rayons X, **caractérisé en ce que** l'au moins un élément fonctionnel (30, 30') présente, au moins dans la zone de sa surface, par laquelle elle est reliée avec le corps de base, une première couche (32), laquelle contient principalement au moins un oxyde métallique et où la première couche a été créée avant la fixation de l'élément fonctionnel sur le corps de base au moyen d'un traitement thermique, ou d'un traitement de recuit par induction électrique, ou au moyen d'un traitement chimique par plasma de l'élément fonctionnel.

2. Implant selon la revendication 1, **caractérisé en ce que** le corps de base présente au moins du magnésium ou un alliage de magnésium en tant que constituant principal.

3. Implant selon l'une des revendications précédentes, **caractérisé en ce que** l'au moins un élément fonctionnel (30, 30') contenant un métal ou un alliage métallique présente au moins un métal du groupe comprenant les éléments tungstène, tantale et titane, ainsi qu'au moins un oxyde du groupe comprenant les composés d'oxydes de tungstène, d'oxydes de tantale et d'oxydes de titane dans sa première couche.

4. Implant selon l'une des revendications précédentes, **caractérisé en ce que** l'au moins un élément fonctionnel (30, 30') présente une enveloppe (35) comprenant un métal ou un alliage métallique contenant au moins un métal du groupe comprenant les éléments tungstène, tantale et titane, ainsi qu'une partie centrale (36) comprenant de préférence un métal ou un alliage métallique contenant au moins un métal du groupe comprenant les éléments platine et or.

5. Implant selon l'une des revendications précédentes, **caractérisé en ce que** l'épaisseur de la première couche (32) se situe entre environ 1 µm et environ 20 µm, de préférence entre environ 1 µm et environ 10 µm.

6. Implant selon l'une des revendications précédentes, **caractérisé en ce que** l'au moins un élément fonctionnel (30, 30') est fixé sur le corps de base avec un produit adhésif (20), de préférence avec un produit adhésif basé sur un polymère.

7. Procédé de fabrication d'un implant, notamment d'une endoprothèse intraluminale, avec un corps de base qui est constitué au moins partiellement d'un matériau métallique biodégradable, avec l'étape suivante :
fixation d'au moins un élément fonctionnel (30, 30') sur le corps de base, qui présente dans une partie de son volume une composition de matériau différente comparativement à celle du corps de base, qui comprend de préférence un matériau radio-opaque et/ou opaque aux rayons X, où l'au moins un élément fonctionnel (30, 30') présente, au moins dans sa zone de surface, par laquelle il est relié avec le corps de base, une première couche (32), laquelle contient essentiellement au moins un oxyde métallique, et où la première couche a été réalisée avant la fixation de l'élément fonctionnel sur le corps de base au moyen d'un traitement thermique, ou d'un traitement de recuit par induction électrique, ou au moyen d'un traitement chimique par plasma de l'élément fonctionnel.

8. Procédé selon la revendication 7, **caractérisé en ce que** l'au moins un élément fonctionnel (30, 30') est fixé sur le corps de base par un collage, de préférence au moyen d'un produit adhésif à base d'un polymère.

9. Procédé selon lune des revendications 7 à 8, **caractérisé en ce que** l'élément fonctionnel est déparé par un produit semi-fini (50) en forme de fil ou de baguette, où le produit semi fini (50) présente de préférence une enveloppe comprenant un métal ou un alliage métallique contenant au moins un métal du groupe constitué par du tungstène, du tantale et du titane, ainsi qu'une partie centrale (36) comprenant de préférence un métal ou un alliage métallique contenant au moins un métal du groupe contenant le platine et l'or.

10. Procédé selon la revendication 9, **caractérisé en ce que** le produit semi-fini (50) en forme de fil ou de baguette est muni de préférence avant la fixation sur le corps de base et/ou de préférence avant la réalisation de la première couche, d'un point de rupture souhaitée (60).

11. Procédé selon la revendication 10, **caractérisé en ce que** l'au moins un point de rupture souhaitée (60) est rendu plus profond pendant une étape de procédé réalisée sur le produit semi fini (50) qui est réalisée avant la fixation de l'élément fonctionnel sur le corps de base pour la formation de la première couche (32).

12. Procédé selon l'une des revendications 7 à 11, **caractérisé en ce que** la fixation de l'élément fonctionnel (30, 30') est effectuée sur le corps de base après une découpe au laser du corps de base.

13. Procédé selon l'une des revendications 7 à 12, **caractérisé en ce que** l'implant est soumis à un traitement chimique par plasma, et/ou à un électro-polissage, après la fixation de l'élément fonctionnel (30, 30') sur le corps de base.

14. Procédé selon la revendication 13, **caractérisé en ce que** le traitement chimique par plasma est effectué **en ce qu'**une tension pulsée est appliquée sur le corps de base, dont l'amplitude dépasse la tension dans le bain caractéristique pour le matériau du corps de base dans une partie du processus de traitement et augmente de préférence au cours du traitement.

15. Procédé selon l'une des revendications 7 à 14, **caractérisé en ce que** l'implant est muni d'une deuxième couche présentant un polymère dégradable, de préférence au moyen d'une pulvérisation, de préférence après le traitement chimique par plasma.
